(19) Europäisches Patentamt
European Patent Office
Office européen des brevets

(11) Veröffentlichungsnummer: **0 280 269 B1**

(12) **EUROPÄISCHE PATENTSCHRIFT**

(45) Veröffentlichungstag der Patentschrift: **12.02.92**

(51) Int. Cl.5: **C07D 311/58**, C07D 335/06, A61K 31/35, A61K 31/38

(21) Anmeldenummer: **88102716.3**

(22) Anmeldetag: **24.02.88**

(54) **3-Amino-dihydro-[1]-benzopyrane und Benzothiopyrane.**

(30) Priorität: **27.02.87 US 20054**

(43) Veröffentlichungstag der Anmeldung:
**31.08.88 Patentblatt 88/35**

(45) Bekanntmachung des Hinweises auf die Patenterteilung:
**12.02.92 Patentblatt 92/07**

(84) Benannte Vertragsstaaten:
**AT BE CH DE ES FR GB GR IT LI LU NL SE**

(56) Entgegenhaltungen:
**EP-A- 0 222 996**

**CHEMICAL ABSTRACTS, Band 107, Nr. 21, 23. November 1987, Seite 60, Zusammenfassung Nr. 190822t, Columbus, Ohio, US; J.M. COSSERY et al.: "The selective labelling of central-5-HT1A receptor binding sites by (3H)5-methoxy-3-(di-n-propylamino)chroman", & EUR. J. PHARMACOL. 1987, 140(29, 143-155**

(73) Patentinhaber: **CIBA-GEIGY AG**
**Klybeckstrasse 141**
**CH-4002 Basel(CH)**

(72) Erfinder: **Hutchison, Alan J.**
**35 Lynwood Road**
**Verona New Jersey 07044(US)**

(74) Vertreter: **Zumstein, Fritz, Dr. et al**
**Bräuhausstrasse 4**
**W-8000 München 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

CHEMICAL ABSTRACTS, Band 92, Nr. 7, 18. Februar 1980, Seite 111, Zusammenfassung Nr. 52486g, Columbus, Ohio, US; N. SARDA et al.: "Effects of 3-amino chroman derivatives on the synthesis index, the capture and spontaneous efflux on catecholamines and serotonin in different regions of the rat brain", & EUR. J. MED. CHEM.-CHIM. THER. 1976, 11(3), 257-262

CHEMICAL ABSTRACTS, Band 85, Nr. 21, 22. November 1976, Seite 34, Zusammenfassung Nr. 153938s, Columbus, Ohio, US; N. SARDA et al.: "Parmacological and biochemical study of 3-aminochromans", & EUR. J. MED. CHEM.-CHIM. THER. 1976, 11(3), 257-262

CHEMICAL ABSTRACTS, Band 101, Nr. 15, 8. Oktober 1984, Seite 697, Zusammenfassung Nr. 130563a, Columbus, Ohio, US; A.S. HORN et al.: "6,7-Dihydroxy-3-chromanamine: synthesis and pharmacological activity of an oxygen isostere of the dopamine agonist", & J. MED. CHEM. 1984, 27(10), 1340-1343

CHEMICAL ABSTRACTS, Band 82, Nr. 5, 3. Februar 1975, Seite 489, Zusammenfassung Nr. 31208w, Columbus, Ohio, US; N. SARDA et al.: "Synthesis and stereochemistry of 3-amino-4-chromanols and 3-aminochromans", & C.R. HEBD. SEANCES ACAD. SCI., SER. C 1974, 279(7), 281-284

## Beschreibung

Die vorliegenden Erfindung bezicht sich auf 3,4-Dihydro-2H-[1]-benzopyran-3-amine, welche als Modulatoren für die Rezeptoren des zentralen Nervensystems verwendbar sind, insbesondere als SerotoninrezeptorAgonisten, beispielsweise für die Behandlung von Störungen des zentralen Nervensystems, insbesondere Depressionen und Angstzuständen, Verfahren zur Herstellung dieser Verbindungen, pharmazeutische Zusammensetzungen, welche diese Verbindungen enthalten, Methoden zur Stimulierung von Serotoninrezeptoren in Säugetieren (einschliesslich Menschen) und zur Behandlung von Syndromen, Zuständen und Krankheiten in Säugetieren durch Anwendung der genannten Verbindungen oder einer pharmazeutischen Zusammensetzung enthaltend die genannten Verbindungen.

Gegenstand der Erfindung sind (+)- und (-)-Enantiomere der 3,4-Dihydro-2H-[1]-benzopyran-3-amin-Derivate der Formel

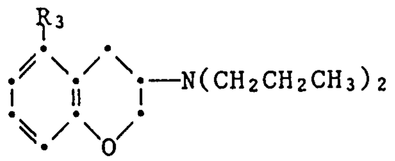

(I)

worin $R_3$ Hydroxy, Methoxy oder Ethoxy bedeutet, und pharmazeutisch annehmbare Salze dieser Verbindungen.

In der Europäischen Patentanmeldung EP-A-0 222 996, die sich auf eine frühere Priorität stützt, aber erst nach dem Prioritätstag der vorliegenden Anmeldung veröffentlicht worden ist und daher als Stand der Technik gemäss EPUe Art. 54(3) gilt, ist die Herstellung von 3,4-Dihydro-2H-[1]-benzopyran-3-aminen und 3,4-Dihydro-2H-[1]-benzothiopyran-3-aminen und deren Verwendung als Modulatoren für Rezeptoren des zentralen Nervensystems beschrieben. Die europäische Patentanmeldung EP-A-0 279 150, die für die Vertragsstaaten ES und GR ebenfalls als Stand der Technik gemäss EPUe Art. 54(3) gilt, beschreibt (+)- und (-)-Enantiomere der Verbindung der Formel I, worin $R_3$ Hydroxy bedeutet.

Der im folgenden verwendete Ausdruck "Nieder" bedeutet, dass so bezeichnete organische Reste oder Verbindungen bis einschliesslich 7 und vorzugsweise bis einschliesslich 4 C-Atome enthalten.

Pharmazeutisch annehmbare Salze sind therapeutisch verwendbare Säureadditionssalze, vorzugsweise Salze von anorganischen oder organischen Säuren, z.B. starken Mineralsäuren, z.B. Halogenwasserstoffsäuren, z.B. Chlorwasserstoff oder Bromwasserstoff, Schwefel-, Phosphor- oder Salpetersäure, aliphatischen oder aromatischen Carbonsäuren oder Sulfonsäuren, z.B. Ameisen-, Essig-, Propion-, Bernstein-, Glycol-, Milch-, Apfel-, Wein-, Zitronen-, Malein-, Fumar-, Brenztrauben-, Phenylessig-, Benzoe-, 4-Aminobenzoe-, Anthranil-, 4-Hydroxybenzoe-, Salicyl-, 4-Amino-salicyl-, Glucon-, Pamoa-, Nicotin-, Methansulfon-, Ethansulfon-, Hydroxyethansulfon-, Benzolsulfon-, p-Toluolsulfon-, Naphthalinsulfon-, Sulfanil-, Cyclohexylsulfamin- oder Ascorbinsäure.

Die neuen Verbindungen der Formel I sind in bekannten pharmakologischen in vitro- und in vivo-Modellen wirksam, welche eine Wirkungsbeziehung hinsichtlich Wirksamkeit bei der Behandlung von Störungen des zentralen Nervensystems bei Säugetieren, vor allem beim Menschen herstellen.

Die erfindungsgemässen Verbindungen sind als selektive Serotonin-(S)-Rezeptor-Stimulatoren (Agonisten) in Säugetieren wirksam, insbesondere mit Selektivität auf den S-1 Rezeptor und vor allem den S-1A Rezeptor.

Daher besitzen die Verbindungen der Formel I wertvolle pharmakologische Eigenschaften in Säugern, insbesondere Serotonin-(S)-Rezeptor stimulierende (agonistische) Eigenschaften, welche für anxiolytische und psychostimulierende Aktivität indiziert ist.

Die genannten Eigenschaften lassen sich in in vitro und in vivo Versuchen, vorzugsweise bei Säugetieren, z.B. Ratten, Hunden oder Affen, aber auch an entnommenen isolierten Organen, Gewebsproben oder enzymatischen Aufbereitungen nachweisen. Die Verbindungen können in vitro in Form von Lösungen, z.B. wässrigen Lösungen, und in vivo entweder enteral oder parenteral, mit Vorteil oral oder intravenös, in Gelatinekapseln, als Stärkesuspensionen oder in wässrigen Lösungen, appliziert werden. Der Dosisbereich in vitro kann zwischen $10^{-4}$ und $10^{-9}$ molaren Konzentrationen betragen. Der Dosisbereich in vivo kann zwischen 0,05 und 30 mg/kg/Tag, vorzugsweise zwischen 0,10 und 1 mg/kg/Tag betragen.

Die Serotonin-(S-1A)-Aufnahme, welche für Regulatoren mit Rezeptoreigenschaften bei Serotonin-Agonisten indiziert ist, kann in vitro wie folgt in einem Bindungsassay nachgewiesen werden:

Man stellt Suspensionen aus Membranmaterial von Rattenhirn her und inkubiert diese mit $^3$H-Serotonin gemäss der Vorschrift von Martin und Sanders-Bush, Naunyn-Schmiedeberg's Arch. Pharmacol. 321, 165 (1982) und Middlemiss und Fozard, Eur. J. Pharmacol., 90, 151 (1983).

In diesem Bindungsassay werden aliquote Teile von 85 ml Zellsuspensionen zu abgepufferten $^3$H-Serotonin-Lösungen mit und ohne Wirkstoff gegeben. Die Konzentration an $^3$H-Serotonin ist 2 nM und man wertet die zu testenden Wirkstoffe über einen weiten Konzentrationsbereich aus. Die $IC_{50}$-Werte (Konzentrationen an Wirkstoff, welche zur Hemmung von 50 % 2 nM $^3$H-Serotonin nötig sind) werden graphisch bestimmt.

So hat beispielsweise die Verbindung 5-Ethoxy-N,N-dipropyl-3,4-dihydro-2H-[1]-benzopyran-3-amin-hydrochlorid einen $IC_{50}$-Wert von ca. $4{,}0 \times 10^{-8}$.

Die Serotonin-Rezeptor-Eigenschaften können auch in vivo durch Abnahme der 5-Hydroxytryptophan-Konzentration in der Hirnrinde von Ratten nach Applikation einer Testverbindung gemäss der in J. Med. Ghem. 21, 864 (1978) beschriebenen Methodik bestimmt werden.

Die besagten vorteilhaften Eigenschaften belegen die nützliche therapeutische Verwendbarkeit der erfindungsgemässen Verbindungen in Säugern (inkl. Menschen) als Arzneimittel mit Wirkung auf das zentrale Nervensystem. Die erfindungsgemässen Verbindungen können in erster Linie als Serotonin-Agonisten mit psychostimulierender Wirkung zur Behandlung von Depressionen, Wahrnehmungsstörungen (senile Demenz) und Kleinhirnstörungen, als Anxiolytika bei der Behandlung von Angstzuständen und als Appetitzügler verwendet werden.

Die erfindungsgemässen Verbindungen sind nach folgenden Verfahren herstellbar:

a) Kondensationsreaktion einer Verbindung der Formel

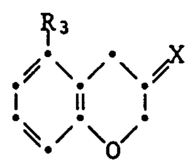

(II),

worin $R_3$ die weiter vorn genannten Bedeutungen hat und X Sauerstoff oder reaktives, verestertes Hydroxy zusammen mit Wasserstoff bedeuten, mit Di-n-propylamin, oder

b) Alkylierung einer Verbindung der Formel

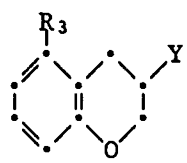

(IV),

worin $R_3$ die weiter vorn genannten Bedeutungen hat und Y $-NH_2$ oder $-NH(CH_2CH_2CH_3)$ darstellt, mit einem reaktionsfähigen Ester von n-Propanol oder mit n-Propionaldehyd unter Einhaltung von reduzierenden Bedingungen, oder

c) Spaltung einer Verbindung der Formel

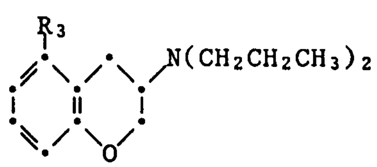

(VI),

worin $R_3$ verethertes Hydroxy bedeutet, zur Herstellung einer Verbindung, worin $R_3$ Hydroxy bedeutet, und/oder Umwandlung einer Verbindung der Formel I, worin $R_3$ Hydroxy bedeutet, durch Veretherung in eine Verbindung der Formel I, worin $R_3$ Methoxy oder Ethoxy bedeutet, oder

d) Reduktion einer Verbindung der Formel

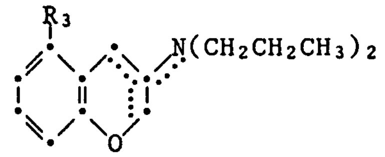

(VII),

worin $R_3$ die weiter vorn genannten Bedeutungen hat, und die punktierten Bindungslinien eine Doppelbindung in einer der angezeigten Positionen darstellen, oder
e) Reduktion einer Verbindung der Formel

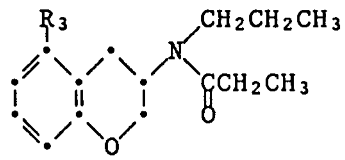

(VIII),

worin $R_3$ die weiter vorn genannten Bedeutungen hat,
wobei man gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein Racemat in die optischen Antipoden aufspaltet.

Eine reaktionsfähige, veresterte Hydroxygruppe in den genannten Verfahrensvarianten ist beispielsweise eine Hydroxygruppe, welche durch eine starke Säure, insbesondere eine starke anorganische Säure wie Halogenwasserstoff, z.B. Chlor-, Brom- oder Jodwasserstoff, oder Schwefelsäure, oder durch eine starke organische Säure, beispielsweise eine Sulfonsäure, wie eine aliphatische oder aromatische Sulfonsäure, z.B. Methansulfonsäure, p-Toluolsulfonsäure oder 4-Bromobenzolsulfonsäure, verestert ist. Eine solche reaktionsfähige, veresterte Hydroxygruppe ist insbesondere Halogen, z.B. Chlor, Brom oder Jod, oder durch aliphatische oder aromatische Gruppen substituiertes Sulfonyloxy, z.B. Mesyloxy oder Tosyloxy.

$R_3$ mit der Bedeutung "verethertes Hydroxy" in der Verfahrensvariante c) ist beispielsweise Niederalkoxy, z.B. Methoxy oder Ethoxy, am Phenylring gegebenenfalls durch diverse Substituenten wie Niederalkyl, z.B. Methyl, Halogen, z.B. Chlor, oder Niederalkoxy, z.B. Methoxy, substituiertes Benzyloxy oder Pyridylniederalkoxy, z.B. Pyridylmethoxy.

Die in den Ausgangsmaterialien, Zwischenstufen und in Verbindungen der Formel I vorhandenen funktionellen Gruppen, insbesondere die Amino- oder Hydroxygruppen, sind gegebenenfalls durch solche Schutzgruppen (conventional protecting groups) geschützt, die üblicherweise in der Penicillin-, Cephalosporin- und Peptidchemie verwendet werden. Diese Schutzgruppen sollen die betreffenden funktionellen Gruppen gegen unerwünschte Nebenreaktionen wie Acylierungen, Veretherungen, Veresterungen, Oxydationen, Solvolyse etc. schützen und unter schonenden Reaktionsbedingungen leicht abspaltbar sein, das heisst unter Vermeidung von Spaltung z.B. solvolytischer, reduktiver, photolytischer oder auch enzymatischer Spaltung, sowie anderer Nebenreaktionen.

Der Schutz von funktionellen Gruppen mit solchen Schutzgruppen, die Schutzgruppen selbst, sowie ihre Abspaltungsreaktionen, sind beispielsweise in "Protective Groups in Organic Chemistry", Plenum Press, London und New York 1973, in Greene, Th.W. "Protective Groups in Organic Synthesis", Wiley, New York 1981, in "The Peptides", Vol. I, Schröder and Lubke, Academic Press, London und New York 1965, sowie in "Methoden der organischen Chemie", Houben-Weyl, 4. Auflage, Bd. 15/I Georg Thieme Verlag, Stuttgart 1974, beschrieben.

Die Herstellung von Verbindungen der Formel I gemäss Verfahrensvariante a) kann man analog bekannten N-Alkylierungsreaktionen durchführen.

Die Herstellung von Verbindungen der Formel I durch reduktive N-Alkylierung von Ausgangsmaterialien, worin X Oxo bedeutet, kann man analog bekannten Reaktionsbedingungen, z.B. mit Hydridreduktionsmitteln wie Natriumcyanborhydrid, durchführen. Die reduktive Aminierung mit Hydridreduktionsmitteln wird vorzugsweise in inertem Lösungsmittel wie Methanol oder Acetonitril, bevorzugt in Gegenwart einer Säure wie Chlorwasserstoffsäure oder Essigsäure, durchgeführt.

Die Herstellung von Verbindungen der Formel I gemäss Verfahrensvariante a) aus Augsgangsmaterialien, worin X eine reaktionsfähige, veresterte Hydroxygruppe und gleichzeitig Wasserstoff darstellt, führt man gegebenenfalls in Gegenwart von Basen wie Triethylamin oder Kaliumcarbonat in einem inerten Lösungsmittel analog bekannten Reaktionsbedingungen für N-Alkylierungen durch.

Die Herstellung von Verbindungen der Formel I gemäss Verfahrensvariante b) wird unter den für die Verfahrensvariante a) genannten Reaktionsbedingungen durchgeführt, beispielsweise bei N-Alkylierungen, wenn das Ausgangsmaterial ein reaktiver Ester von n-Propanol ist, oder N-Alkylierungen unter reduzierenden Bedingungen, wenn das Ausgangsmaterial n-Propionaldehyd ist.

Die Herstellung von Verbindungen der Formel I, worin $R_3$ Hydroxy ist, gemäss Verfahrensvariante c) aus Ausgangsmaterialien der Formel VI, worin $R_3$ verethertes Hydroxy darstellt, erfolgt in an sich bekannter Weise, z.B. in Gegenwart einer Mineralsäure wie Jodwasserstoff, und zwar bevorzugt aus solchen Ausgangsmaterialien, worin $R_3$ Niederalkoxy, z.B. Methoxy, ist, durch Umsetzung mit Natrium- oder Lithiumdiphenylphosphid in Tetrahydrofuran unter Rückflusstemperatur oder mit Bortribromid in Methylenchlorid.

Bei Verwendung von Ausgangsmaterialien der Formel VI, worin $R_3$ gegebenenfalls substituiertes Benzyloxy bedeutet, führt man die Herstellung der Endstoffe gemäss Verfahrensvariante c) vorzugsweise unter den bekannten Bedingungen der katalytischen Hydrierung, beispielsweise mit Palladium als Katalysator durch.

Die Herstellung von Verbindungen der Formel I gemäss Verfahrensvariante d) führt man unter solchen Reaktionsbedingungen durch, welche für Reaktionen, in denen Kohlenstoff-Stickstoff-Doppelbindungen oder Kohlenstoff-Kohlenstoff-Doppelbindungen in einer Enamin-Gruppierung gesättigt werden, bekannt sind, z.B. durch Umsetzung mit Hydridreduktionsmitteln wie Lithiumaluminiumhydrid oder einem Boran, Natriumborhydrid, Natriumcyanoborhydrid oder Diboran, in inerten Lösungsmitteln wie THF oder Diethylether, vorzugsweise bei Raumtemperatur oder unter Erwärmen.

Ausgangsmaterialien der Formel II sind bekannt oder können nach bekannten Verfahren hergestellt werden.

Ausgangsmaterialien der Formel II, d.h. die Chroman-3-one, können nach an sich bekannten Verfahren hergestellt werden, z.B. wie in J. Chem. Soc. 1610 (1948), J. Amer. Chem. Soc. 87, 3958 (1965) und in Bull. Soc. Chim. Belg. 82, 283 (1973) oder wie in den Bespielen beschrieben.

Beispielsweise wird gemäss der in Bull. Soc. Chim. Belg. 82, 283 (1973) beschriebenen Methode ein durch Niederalkoxy oder Benzyloxy substituiertes Salicylaldehyd mit einem Acrylsäureniederalkylester kondensiert und das entsprechend substituierte 2H-[1]-Benzopyran-3-carbonsäure-Derivat erhalten. Curtius-Abbau zum 3-Amino-2H-[1]-benzopyran-Derivat und saure Hydrolyse ergibt die entsprechend substituierten Chroman-3-on-Derivate.

Ausgangsmaterialien der Formel II, können in vorteilhafter Weise gemäss folgendem neuen Verfahren wie folgt hergestellt werden:

Ein durch Niederalkoxy oder Benzyloxy substituiertes 2H-[1]-benzopyran, z.B. eine Verbindung wie in J. Org. Chem. 38, 3832 (1973) beschrieben, wird mit einem Halogenierungsmittel, z.B. mit N-Bromosuccinimid oder mit N-Bromoacetamid, in einem Hydroxylgruppen-haltigen Lösungsmittel, z.B. in wässrigem Aceton, umgesetzt, worauf man das entsprechend substituierte 3-Halo-4-hydroxychroman erhält. Durch Umsetzung mit einer nicht-wässrigen Base, z.B. Natriumhydrid in Tetrahydrofuran, erhält man das entsprechend substituierte Chroman-3,4-epoxid. Nach Umlagerung in einem Lewis-Säurehaltigen Reagenz, z.B. Zinkjodid in Toluol, erhält man die entsprechend durch Niederalkoxy oder Benzyloxy substiutierte Zwischenstufe.

Die Hydroxy-substituierten Chroman-3-one werden vorzugsweise durch Etherspaltung der entsprechenden Niederalkylether oder Benzylether, vorzugsweise durch Spaltung der Methylether mit für Etherspaltungen bekannten Reagenzien, insbesondere mit Lithiumdiphenylphosphid in Tetrahydrofuran unter Rückflussbedingungen, erhalten.

Die durch Acyloxygruppen substituierten 3,4-Dihydro-2H-[1]-benzopyran-3-one können durch Acylierung der entsprechenden Hydroxy-substituierten 3,4-Dihydro-2H-[1]-benzopyran-3-one analog bekannten Acylierungsverfahren hergestellt werden.

Die primären und sekundären 3-Amino-3,4-dihydro-2H-[1]-benzopyran-Ausgangsmaterialien der Formel IV können aus Ausgangsmaterialien der Formel II mit der Bedeutung X = Oxo durch Umsetzung mit Ammoniak, n-Propylamin oder vorzugsweise mit einem Säureadditionssalz davon in Gegenwart eines Reduktionsmittels, vorzugsweise mit Natriumcyanborhydrid hergestellt werden.

Primäre 3-Amino-3,4-dihydro-2H-[1]-benzopyran-Ausgangsmaterialien der Formel IV, welche für die Verfahrensvariante b) verwendbar sind, können ebenfalls durch Umsetzung eines gegebenenfalls substituierten Phenolderivats mit alpha-Halomethylacrylsäure und anschliessender Zyklisierung der alpha-Phenoxymethylacrylsäure bei erhöhter Temperatur zur gegebenenfalls substituierten 3,4-Dihydro-2H-[1]-benzopyran-3-carbonsäure hergestellt werden. Nach Curtius-Abbau durch Umsetzung mit Diphenylphosphonylazid erhält man die entsprechend substituierten 3,4-Dihydro-3-amino-2H-[1]-benzopyran-Derivate.

Die Zwischenprodukte der Formel VI, welche in der Verfahrensvariante c) zur Anwendung gelangen, lassen sich beispielsweise durch reduktive Aminierung von entsprechend substituierten 3,4-Dihydro-2H-[1]-benzopyran-3-on-Derivaten mit Di-n-propylamin analog den weiter vorn beschriebenen Reaktionsbedingungen herstellen.

Zwischenprodukte der Formel VII können durch Behandlung der entsprechend substituierten 3,4-Dihydro-2H-[1]-benzopyran-3-one mit Di-n-propylamin unter Reaktionsbedingungen, welche durch Wasserentzug gekennzeichnet sind, z.B. in Gegenwart von Molekularsieb, Bortrifluoridetherat oder p-Toluolsulfonäsure, in einem inerten Lösungsmittel wie Toluol oder Methylenchlorid hergestellt werden.

Zwischenprodukte der Formel VIII können durch Acylierung einer Verbindung der Formel IV, worin Y $-NH(CH_2CH_2CH_3)$ darstellt, mit Propionsäure in Gegenwart eines gängigen Kondensationsmittels wie N,N-Dicyclohexylcarbodiimid oder durch Umsetzung mit einem reaktionsfähigen, funktionellen Säurederivat, z.B. dem Säurechlorid, oder einem gemischten Anhydrid, welches man durch Reaktion der Säure mit einem Halogenameisensäureester wie Chlorameisensäureethylester, in einem inerten, gegebenenfalls wasserfreien Lösungsmittel wie Methylenchlorid bildet, vorzugsweise in Gegenwart einer Base wie Pyridin, hergestellt werden.

Erfindungsgemässe Verbindungen, die nach irgendeiner der weiter vorn beschriebenen Verfahrensvarianten erhalten werden, können analog bekannten Verfahren, wie zum Beispiel im folgenden Abschnitt erläutert wird, in andere erfindungsgemässe Verbindungen der Formel I umgewandelt werden.

Die Umwandlung von Verbindungen der Formel I, worin $R_3$ Hydroxy bedeutet, in Verbindungen, worin $R_3$ Methoxy oder Ethoxy bedeutet, kann bevorzugt durch Kondensation mit der äquivalenten Menge des reaktionsfähigen Esters von Methanol oder Ethanol, z.B. einer Halogen-Verbindung, wie einem Methyl- oder Ethyliodid, in Gegenwart von äquivalenten Mengen von starken Basen wie Natriumhydrid in einem nichtwässrigen Lösungsmittel, wie Dimethylsulfoxid oder Dimethylformamid, erfolgen.

In allen obigen Reaktionen, kann es vorteilhaft sein, potentiell reaktionsfähige, z.B. die Hydroxy- oder Aminogruppe oder andere störende Gruppen, gemäss an sich bekannten Methoden zu schützen. Man schützt solche Gruppen vor der gewünschten Reaktion und, wenn erwünscht, spaltet nachträglich die Schutzgruppen ab, wobei man die gewünschten Verbindungen, z.B. solche der Formel I oder Zwischenprodukte erhält.

Eine Hydroxygruppe kann in Form von Estern, z.B. als Acyloxy-derivate, z.B. als Niederalkanoyl-, Benzyloxycarbonyl- oder Niederalkoxycarbonylester, oder in Form von Ethern, z.B. als Tetrahydropyranyl- oder Benzylether, geschützt werden. In einer erhaltenen geschützten Verbindung der Formel I oder in einem Zwischenprodukt, worin eine oder mehrere funktionelle Gruppen geschützt sind, können die geschützten funktionellen Gruppen, z.B. die Hydroxy- oder Aminogruppen, nach an sich bekannten Methoden, z.B. Solvolyse, insbesondere Hydrolyse mit einer Säure, oder durch Reduktion, insbesondere Hydrogenolyse, freigesetzt werden.

Die oben genannten nachträglichen Reaktionen werden nach an sich bekannten Methoden, in Gegenwart oder Abwesenheit von Verdünnungsmitteln, vorzugsweise solche, welche gegenüber den Reagenzien inert sind und diese lösen, Katalysatoren, Kondensations- oder anderen oben genannten Mitteln, und/oder in einer inerten Atmosphäre, unter Kühlung, bei Zimmertemperatur oder bei erhöhter Temperatur, vorzugsweise beim Siedepunkt des verwendeten Lösungsmittels, bei normalem oder erhöhtem Druck, durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder wonach ein Ausgangsmaterial unter den Reaktionsbedingungen gebildet, oder worin ein Ausgangsstoff in Form eines Salzes oder optisch reinen Antipoden verwendet wird. Immer wenn gewünscht, werden die oben genannten Verfahren erst durchgeführt, nachdem alle potentiell störenden, reaktionsfähigen funktionellen Gruppen in geeigneter Weise geschützt sind, z.B. wie oben und in den Beispielen illustriert.

In den genannten Reaktionen werden vorteilhafterweise solche Ausgangsstoffe vewendet, welche zu den weiter vorn als besonders bevorzugt beschriebenen Verbindungen führen.

Erhaltene racemische Produkte können in die optischen Antipoden gespalten werden, z.B. durch Trennung ihrer diastereomeren Salze, wie gemäss J. Org. Chem. 43, 3803 (1978), z.B. durch fraktionierte Kristallisation von d- und ℓ-(Tartraten, Mandelaten, Camphersulfaten oder 1-Naphthyl-1-ethylisocyanaten) von Verbindungen, die eine basische, salzbildende Gruppe haben, oder von d- und ℓ-($\alpha$-Methylbenzylamin, Cinchonidin, Chinin, Chinidin, Ephedrin, Dehydroabietylamin, Brucin oder Strychnin)-Salzen von Verbindungen, die eine saure,salzbildende Gruppe besitzen.

Bevorzugt wird jeweils der wirksamere Antipode der erfindungsgemässen Verbindungen isoliert.

Die Verbindungen der vorliegenden Erfindung werden entweder in freier oder in Form ihrer Salze erhalten. Jede erhaltene Base kann in das entsprechende Säureadditionssalz umgewandelt werden, vorzugsweise unter Verwendung einer therapeutisch annehmbaren Säure oder eines Anionenaustauschers. Erhaltene Salze können in die entsprechenden freien Basen umgewandelt werden, zum Beispiel unter Verwendung einer stärkeren Base, beispielsweise eines Metall- oder Ammoniumhydroxids oder eines basischen Salzes, z.B. eines Alkalimetallhydroxids oder -carbonats, oder eines Kationenaustauschers. Diese oder andere Salze, etwa Pikrate, können auch zur Reinigung der erhaltenen Basen verwendet werden, indem man die Basen in Salze überführt, diese abtrennt und reinigt, und aus den Salzen wiederum die Base freisetzt. Infolge der engen Beziehung zwischen den freien Verbindungen und ihren Salzen sind in diesem Zusammenhang unter freien Verbindungen sinn- und zweckmässig gegebenenfalls immer auch die entsprechenden Salze zu verstehen.

Die Verbindungen und ihre Salze können auch in Form ihrer Hydrate erhalten werden, oder sie können andere zur Kristallisation verwendete Lösungsmittel einschliessen.

Die vorliegende Erfindung betrifft ebenfalls Verbindungen der Formel I sowie pharmazeutisch annehmbare Salze, insbesondere nicht-toxische Säureadditionssalze davon oder pharmazeutische Zusammensetzungen zur therapeutischen Verwendung in Säugetieren (inkl. Menschen) als Heilmittel, insbesondere neurotropische Heilmittel zur Behandlung von Störungen des zentralen Nervensystems als Folge von Stimulierungserscheinungen der Serotoninrezeptoren, z.B. Depressionen und Angstzuständen, zum Gegenstand.

Die vorliegende Erfindung hat ebenfalls die Verwendung von Verbindungen der Formel I zur Herstellung von pharmazeutischen Zusammensetzungen, insbesondere pharmazeutischen Zusammensetzungen mit stimulierender Wirkung auf Serotoninrezeptoren, zum Gegenstand, welche sich beispielsweise für die Behandlung von Depression und Angstzuständen eignen.

Die erfindungsgemässen pharmazeutischen Präparate eignen sich zur enteralen, wie oralen oder rektalen, transdermalen oder parenteralen Verabreichung an Säugetieren, inkl. Menschen, zur Behandlung von Krankheiten, die in Folge von Serotoninrezeptorstimulierung, z.B. Depressionen und Angstzuständen, auftreten. Diese Präparate enthalten eine wirksame Menge einer Verbindung der Formel I oder eines pharmazeutisch annehmbaren Salzes davon und zwar ohne Beimengung oder in Kombination mit einem oder mehreren pharmazeutisch annehmbaren Trägermaterialien.

Die pharmakologisch aktiven Verbindungen der Erfindung sind bei der Herstellung von pharmazeutischen Zusammensetzungen verwendbar, die eine wirksame Menge derselben in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium- oder Calciumsalz und/oder Polyethylenglycol, für Tabletten auch c) Bindemitteln, z.B. Magnesiumaluminiumsilicat, Stärkepaste, Gelatine, Tragant, Methylcellulose, Natriumcarboxymethylcellulose und/oder Polyvinyl-pyrrolidon, gewünschtenfalls d) Zerteilungs- bzw. Desintegrationsmitteln, z.B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvanzien, wie Konservierungs-, Stabilisierungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier- bzw. Ueberzugsmethoden hergestellt und enthalten etwa 0,1 bis 75 %, vorzugsweise etwa 1 bis 50 %, des aktiven Bestandteils.

Geeignete Formulierungen für die transdermale Anwendung enthalten eine wirksame Menge einer Verbindung der Formel I zusammen mit Trägermaterial. Geeignete Trägermaterialien enthalten pharmazeutisch annehmbare Hilfsstoffe, welche den Durchgang durch die Haut ermöglichen. Insbesondere haben transdermale therapeutische Systeme die Form eines Pflasters mit einer Schutzschicht, einem Wirkstoffreservoir, gegebenenfalls mit Trägermaterialien, und einer die Abgabe bestimmenden Kontrollschicht, durch welche der Wirkstoff auf die Haut mit kontrollierter und bestimmbarer Geschwindigkeit über einen längeren Zeitabschnitt abgegeben wird. Das System hat gegebenenfalls noch eine Klebschicht.

Zur Behandlung von Störungen des zentralen Nervensystems in Säugetieren inkl. Menschen als Folge von Stimulierungserscheinungen der Serotoninrezeptoren, z.B. von Angstzuständen, kann beispielsweise eine therapeutisch wirksame Menge einer Verbindung der Formel I oder eines pharmazeutisch annehmbaren Salzes davon angewendet werden.

Die applizierbare Wirkstoffdosis ist speziesabhängig sowie vom Körpergewicht, dem Alter und individuellen Zustand und der Darreichungsform abhängig.

Die Einheitsdosis für einen Säuger, z.B. Mensch, von ca. 50 bis 70 kg Gewicht beträgt zwischen 15 und

200 mg des Wirkstoffs.

Die folgenden Beispiele sollen die Erfindung erläutern und keine Einschränkungen darstellen. Die Temperaturen sind in Celsiusgraden angegeben, und sämtliche Teile sind in Form von Gewichtsteilen angegeben. Wenn nicht anders erwähnt, werden sämtliche Verdampfungsvorgänge unter vermindertem Druck vorzugsweise zwischen etwa 15 und 100 mg Hg durchgeführt.

Beispiel 1:

a) Eine Mischung bestehend aus 4,0 g 5 = Methoxy-3,4-dihydro-2H-[1]-benzopyran-3-on, 30 ml N,N-Di-n-propylamin, 200 ml Toluol und 0,5 ml Trifluoressigsäure wird 3 Stunden in einer Dean-Stark-Apparatur erhitzt. Das Lösungsmittel wird im Vakuum entfernt und der Rückstand zu einer Lösung von 7 g Natriumcyanoborhydrid in 160 ml Ethanol und 40 ml Essigsäure gegeben. Nach 15 Minuten Reaktionszeit bei Raumtemperatur wird das Reaktionsgmemisch mit 6N wässriger Chlorwasserstoffsäure verdünnt, mit Ether gewaschen, mit 45 %iger wässriger Kaliumhydroxidlösung basisch gemacht und mit Ether wiederum extrahiert. Nach dem Trocknen über Magnesiumsulfat wird das Lösungsmittel im Vakuum entfernt. Nach Zugabe von ethanolischer Chlorwasserstofflösung zu dem in Ether aufgenommenen Rückstand erhält man 5-Methoxy-3,4-dihydro-N,N-dipropyl-2H-[1]-benzopyran-3-amin-hydrochlorid mit einem Schmelzpunkt von 219-221°.

Das Ausgangsmaterial wird folgendermassen erhalten:

Zu einer gut gerührten Lösung von 36,4 g 5-Methoxy-2H-[1]-benzopyran in 300 ml Aceton und 100 ml Wasser werden 29 g N-Bromsuccinimid in Portionen über einen Zeitraum von 5 Minuten gegeben. Nach 4 Stunden Reaktionszeit wird das Reaktionsgemisch mit Wasser verdünnt und mit Ether extrahiert. Die vereinigten organischen Phasen werden mit Wasser gewaschen und über Magnesiumsulfat getrocknet. Nach Abziehen des Lösungsmittel wird der Rückstand mit Ether-Hexan verrieben, worauf man trans-3-Brom-4-hydroxy-5-methoxy-3,4-dihydro-2H-[1]-benzopyran erhält.

Zu einer Suspension von 5,0 g Natriumhydrid in 100 ml trockenem Tetrahydrofuran wird tropfenweise unter Rühren eine Lösung von 13 g trans-3-Brom-4-hydroxy-5-methoxy-3,4-dihydro-2H-[1]-benzopyran in 150 ml Tetrahydrofuran gegeben. Nach 30 Minuten Rühren bei Raumtemperatur wird das Reaktionsgemisch filtriert und das Lösungsmittel abgezogen. Nach Lösen des Rückstands in 150 ml Toluol werden 1,0 g wasserfreies Zinkiodid hinzugefügt und 15 Minuten unter Rückflussbedingungen erhitzt. Das Reaktionsgemisch wird durch 120 g Silicagel mit Methylenchlorid als Elutionsmittel filtriert. Nach Abziehen des Lösungsmittels erhält man 5-Methoxy-3,4-dihydro-2H-[1]-benzopyran-3-on (5-Methoxychroman-3-on) als Oel.

Beispiel 2:

a) Ein Gemisch bestehend aus 5,5 g 5-Methoxy-N,N-dipropyl-3,4-dihydro-2H-[1]-benzopyran-3-amin und 3,9 g Dibenzoyl-D-weinsäure in 65 ml Methylethylketon wird erwärmt und anschliessend auf Raumtemperatur abgekühlt. Man filtriert den Feststoff ab und kristallisiert diesen dreimal aus Methylethylketon um. Nach Umwandlung in die freie Base erhält man linksdrehendes (-)-5-Methoxy-N,N-di-n-propyl-3,4-dihydro-2H-[1]-benzopyran-3-amin, $[\alpha]_D^{25} = 74,9°$. Die freie Base lässt sich in (-)-5-Methoxy-N,N-di-n-propyl-3,4-dihydro-2H-[1]-benzopyran-3-amin-hydrochlorid überführen, $[\alpha]_D = -21,79°$ (Methanol), Schmelzpunkt: 144-146°.

b) Mit Dibenzoyl-L-weinsäure lässt sich rechtsdrehendes (+)-5-Methoxy-N,N-di-n-propyl-3,4-dihydro-2H-[1]-benzopyran-3-amin-hydrochlorid, $[\alpha]_D = +19,59°$ (Methanol), Schmelzpunkt: 144-146°, herstellen.

Beispiel 3:

a) Zu einer Lösung von 8,55 ml Diphenylphosphin in 50 ml trockenem Tetrahydrofuran werden 22 ml 2,2M n-Butyllithium in Hexan bei 0° gegeben. Zu dieser Lösung gibt man 7,3 g 5-Methoxy-3,4-dihydro-N,N-dipropyl-2H-[1]-benzopyran-3-amin in etwas Tetrahydrofuran und erhitzt vier Stunden lang unter Rückflussbedingungen. Man verdünnt das Reaktionsgemisch mit Ether und extrahiert mit 3N Salzsäure. Die wässrige Phase wird mit Ether gewaschen, mit wässriger Natriumhydrogencarbonat-Lösung neutralisiert und mit Methylenchlorid extrahiert. Nach Trocknen über Magnesiumsulfat zieht man das Lösungsmittel ab und löst den Rückstand in einem Acetonitril-Ether-Gemisch. Nach Ansäuern mit 3N Salzsäure-Lösung erhält man 5-Hydroxy-3,4-dihydro-N,N-dipropyl-2H-[1] benzopyran-3-amin-hydrochlorid. F.p.: 206-210°.

b) Analog a) wird (-)5-Methoxy-N,N-dipropyl-3,4-dihydro-2H-[1]-benzopyran-3-amin in (-)-5-Hydroxy-N,N-dipropyl-3,4-dihydro-2H-[1]-benzopyran-3-amin-hydrochlorid umgewandelt, $[\alpha]_D$ = -24.84° (Methanol), Schmelzpunkt: 224-227°.
c) Analog erhält man das rechtsdrehende Enantiomere (+)-5-Hydroxy-N,N-dipropyl-3,4-dihydro-2H-[1]-benzopyran-3-amin-hydrochlorid, $[\alpha]_D$ = +23.27° (Methanol), Schmelzpunkt: 225-228°.

Beispiel 4:

a) Zu einer Suspension bestehend aus 55 mg Natriumhydrid in 10 ml Dimethylsulfoxid werden 290 mg 5-Hydroxy-N,N-dipropyl-3,4-dihydro-2H-[1]-benzopyran-3-amin gegeben. Nach 15 Minuten Reaktionszeit werden 180 mg Ethyliodid hinzugefügt und drei Stunden lang gerührt. Man entfernt das Lösungsmittel und verdünnt mit Wasser. Man extrahiert mit Ether und trocknet die organischen Phasen über Magnesiumsulfat. Nach Abziehen des Lösungsmittels wird der Rückstand in Ether aufgenommen und mit 6N ethanolischer Chlorwasserstoff-Lösung angesäuert, worauf man 5-Ethoxy-N,N-dipropyl-3,4-dihydro-2H-[1]-benzopyran-3-amin-hydrochlorid erhält. F.p.: 135-137°.
b) Analog a) wandelt man (-)-5-Hydroxy-N,N-dipropyl-3,4-dihydro-2H-[1]-benzopyran-3-amin in (-)-5-Ethoxy-N,N-dipropyl-3,4-dihydro-2H-[1]-benzopyran-3-amin-hydrochlorid um, $[\alpha]_D$ = -32.88° (Methanol), Schmelzpunkt: 149-150°;
c) Analog wird das rechtsdrehende Enantiomere (+)-5-Ethoxy-N,N-dipropyl-3,4-dihydro-2H-[1]-benzopyran-3-amin-hydrochlorid, $[\alpha]_D$ = +31.91° (Methanol), Schmelzpunkt: 148-149°, erhalten.

Beispiel 5:

a) Herstellung von 10.000 Tabletten mit 10,0 mg Wirkstoff:

| Bestandteile | |
|---|---|
| (+)-3,4-Dihydro-N,N-dipropyl-5-ethoxy-2H-[1]-benzopyran-3-amin-hydrochlorid | 200,00 g |
| Lactose | 2.400,00 g |
| Maisstärke | 125,00 g |
| Polyethylenglycol 6,000 | 150,00 g |
| Magnesiumstearat | 40,00 g |
| Wasser (steril) | q.s. |

Das pulverige Material wird durch ein Sieb mit Oeffnungen von 0,6 mm gedrückt. Anschliessend mischt man in einem Mischer den Wirkstoff mit der Lactose, dem Magnesiumstearat und der halben Menge Stärke. Die andere halbe Menge Stärke wird in 65 ml Wasser suspendiert und diese Suspension zu einer siedenden Lösung des Polyethylenglycols 260 ml in Wasser gegeben. Diese gebildete Paste wird mit der Pulvermischung versetzt, welche gegebenenfalls mit weiterem Wasser granuliert wird. Man trocknet das Granulat bei 35°, treibt dieses durch ein Sieb mit 1,2 mm weiten Oeffnungen und komprimiert zu konkaven Tabletten mit oberer Bruchkerbe.

b) Herstellung von 1.000 Kapseln mit 10,0 mg Wirkstoff:

| Bestandteile | |
|---|---|
| (+)-3,4-Dihydro-N,N-dipropyl-5-ethoxy-2H-[1]-benzopyran-3-amin-hydrochlorid | 10,00 g |
| Lactose | 207,00 g |
| Stärke | 80,00 g |
| Magnesiumstearat | 3,00 g |

Das pulverige Material wird durch ein Sieb mit Oeffnungen von 0,6 mm Durchmesser gedrückt. Anschliessend mischt man in einem Mischer den Wirkstoff mit der Lactose, dem Magnesiumstearat und der Stärke bis man eine homogene Masse erhält. Man füllt Nr. 2 Hartgelatinekapseln mit 300 mg der vorbereiteten Mischung.

Analog kann man Kapseln mit 5 - 100 mg eines anderen von der Formel I definierten Wirkstoffs

herstellen.

**Patentansprüche**
**Patentansprüche für folgende Vertragsstaaten : DE, GB, FR, IT, NL, SE, LI, CH, BE, AT, LU**

**1.** (+)- und (-)-Enantiomere der Verbindungen der Formel

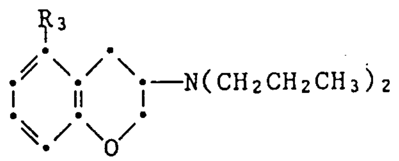

(I)

worin $R^3$ Hydroxy, Methoxy oder Ethoxy bedeutet, und pharmazeutisch annehmbare Salze dieser Verbindungen.

**2.** Das (+)-Enantiomere der Verbindung der Formel I gemäss Anspruch 1, worin $R^3$ Hydroxy bedeutet, und pharmazeutisch annehmbare Salze dieser Verbindung.

**3.** Das (-)-Enantiomere der Verbindung der Formel I gemäss Anspruch 1, worin $R^3$ Hydroxy bedeutet, und pharmazeutisch annehmbare Salze dieser Verbindung.

**4.** Das (+)-Enantiomere der Verbindung der Formel I gemäss Anspruch 1, worin $R^3$ Methoxy bedeutet, und pharmazeutisch annehmbare Salze dieser Verbindung.

**5.** Das (-)-Enantiomere der Verbindung der Formel I gemäss Anspruch 1, worin $R^3$ Methoxy bedeutet, und pharmazeutisch annehmbare Salze dieser Verbindung.

**6.** Das (+)-Enantiomere der Verbindung der Formel I gemäss Anspruch 1, worin $R^3$ Ethoxy bedeutet, und pharmazeutisch annehmbare Salze dieser Verbindung.

**7.** Das (-)-Enantiomere der Verbindung der Formel I gemäss Anspruch 1, worin $R^3$ Ethoxy bedeutet, und pharmazeutisch annehmbare Salze dieser Verbindung.

**8.** (+)-5-Ethoxy-N,N-di-n-propyl-3,4-dihydro-2H-[1]-benzopyran-3-amin-hydrochlorid.

**9.** Pharmazeutische Präparate enthaltend Verbindungen der Ansprüche 1 bis 8 oder ein pharmazeutisch annehmbares Salz davon zusammen mit einem pharmazeutisch geeigneten Trägermaterial.

**10.** Die in den Ansprüchen 1 bis 8 genannten Verbindungen zur Anwendung in einem Verfahren zur therapeutischen Behandlung des menschlichen oder tierischen Körpers.

**11.** Die in den Ansprüchen 1 bis 8 genannten Verbindungen als Serotoninagonisten.

**12.** Verfahren zur Herstellung von Verbindungen gemäss Anspruch 1, dadurch gekennzeichnet, dass man

a) eine Verbindung der Formel

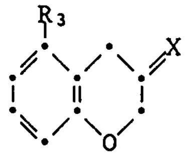

(II),

worin $R_3$ die genannten Bedeutungen hat und X Sauerstoff oder reaktives, verestertes Hydroxy zusammen mit Wasserstoff bedeuten, mit Di-n-propylamin kondensiert, oder

b) eine Verbindung der Formel

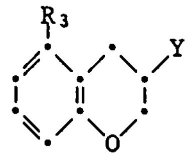

(IV),

worin $R_3$ die genannten Bedeutungen hat und Y $-NH_2$ oder $-NH(CH_2CH_2CH_3)$ darstellt, mit einem reaktionsfähigen Ester von n-Propanol oder mit n-Propionaldehyd unter Einhaltung von reduzierenden Bedingungen alkyliert, oder

c) eine Verbindung der Formel

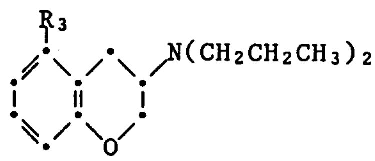

(VI),

worin $R_3$ verethertes Hydroxy bedeutet, zur Herstellung einer Verbindung, worin $R_3$ Hydroxy bedeutet, spaltet und/oder eine Verbindung der Formel I, worin $R_3$ Hydroxy bedeutet, durch Veretherung in eine Verbindung der Formel I, worin $R_3$ Methoxy oder Ethoxy bedeutet, umwandelt, oder

d) eine Verbindung der Formel

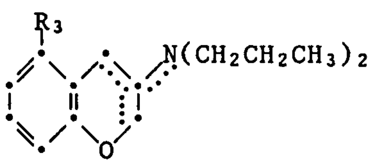

(VII),

worin $R_3$ die genannten Bedeutungen hat und die punktierten Bindungslinien eine Doppelbindung in einer der angezeigten Positionen darstellen, reduziert, oder

e) eine Verbindung der Formel

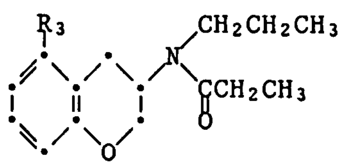

(VIII),

worin $R_3$ die genannten Bedeutungen hat, reduziert,

und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein Racemat in die optischen Antipoden aufspaltet.

**Patentansprüche für folgende Vertragsstaaten : ES, GR**

1. Verfahren zur Herstellung von (+)- und (-)-Enantiomeren der Verbindungen der Formel

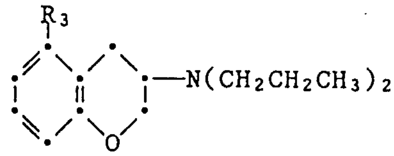

(I)

worin $R_3$ Methoxy oder Ethoxy bedeutet, und von pharmazeutisch annehmbaren Salzen dieser Verbindungen, dadurch gekennzeichnet, dass man

a) eine Verbindung der Formel

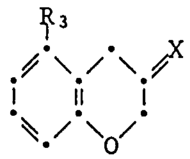

(II),

worin $R_3$ die genannten Bedeutungen hat und X Sauerstoff oder reaktives, verestertes Hydroxy zusammen mit Wasserstoff bedeuten, mit Di-n-propylamin kondensiert, oder
b) eine Verbindung der Formel

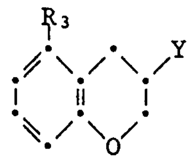

(IV)

worin $R_3$ die genannten Bedeutungen hat und Y $-NH_2$ oder $-NH(CH_2CH_2CH_3)$ darstellt, mit einem reaktionsfähigen Ester von n-Propanol oder mit n-Propionaldehyd unter Einhaltung von reduzierenden Bedingungen alkyliert, oder
c) eine Verbindung der Formel

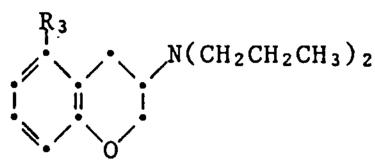

(VI),

worin $R_3$ Hydroxy bedeutet, verethert, oder
d) eine Verbindung der Formel

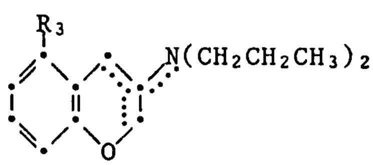

(VII),

worin $R_3$ die genannten Bedeutungen hat und die punktierten Bindungslinien eine Doppelbindung in einer der angezeigten Positionen darstellen, reduziert, oder

e) eine Verbindung der Formel

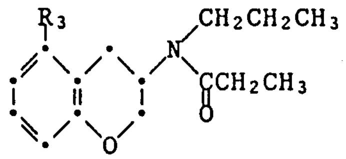

(VIII),

worin $R_3$ die genannten Bedeutungen hat, reduziert,

und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein Racemat in die optischen Antipoden aufspaltet.

**2.** Verfahren gemäss Anspruch 1 zur Herstellung des (+)-Enantiomeren der Verbindung der Formel 1, worin $R^3$ Methoxy bedeutet, und von pharmazeutisch annehmbaren Salzen dieser Verbindung.

**3.** Verfahren gemäss Anspruch 1 zur Herstellung des (-)-Enantiomeren der Verbindung der Formel I, worin $R^3$ Methoxy bedeutet, und von pharmazeutisch annehmbaren Salzen dieser Verbindung

**4.** Verfahren gemäss Anspruch 1 zur Herstellung des (+)-Enantiomeren der Verbindung der Formel I, worin $R^3$ Ethoxy bedeutet, und von pharmazeutisch annehmbaren Salzen dieser Verbindung.

**5.** Verfahren gemäss Anspruch 1 zur Herstellung des (-)-Enantiomeren der Verbindung der Formel I, worin $R^3$ Ethoxy bedeutet, und von pharmazeutisch annehmbaren Salzen dieser Verbindung.

**6.** Verfahren gemäss Anspruch 1 zur Herstellung von (+)-5-Ethoxy-N,N-di-n-propyl-3,4-dihydro-2H-[1]-benzopyran-3-amin-hydrochlorid.

**7.** Verfahren zur Herstellung von pharmazeutischen Präparaten, dadurch gekennzeichnet, dass man eine gemäss Anspruch 1 bis 6 herstellbare Verbindung mit einem Trägermaterial mischt.

**Claims**

**Claims for the following Contracting States : DE, GB, FR, IT, NL, SE, LI, CH, BE, AT, LU**

**1.** A (+)- or (-)-enantiomer of a compound of formula

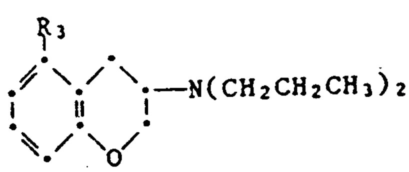

(I)

wherein $R_3$ is hydroxy, methoxy or ethoxy, or a pharmaceutically acceptable salt of that compound.

**2.** The (+)-enantiomer of the compound of formula I according to claim 1 wherein $R_3$ is hydroxy, or a pharmaceutically acceptable salt of that compound.

**3.** The (-)-enantiomer of the compound of formula I according to claim 1 wherein $R_3$ is hydroxy, or a phamaceutically acceptable salt of that compound.

**4.** The (+)-enantiomer of the compound of formula I according to claim 1 wherein $R_3$ is methoxy, or a pharmaceutically acceptable salt of that compound.

**5.** The (-)-enantiomer of the compound of formula I according to claim 1 wherein $R_3$ is methoxy, or a pharmaceutically acceptable salt of that compound.

**6.** The (+)-enantiomer of the compound of formula I according to claim 1 wherein $R_3$ is ethoxy, or a pharmaceutically acceptable salt of that compound.

**7.** The (-)-enantiomer of the compound of formula I according to claim 1 wherein $R_3$ is ethoxy, or a pharmaceutically acceptable salt of that compound.

**8.** (+)-5-ethoxy-N,N-di-n-propyl-3,4-dihydro-2H-[1]-benzopyran-3-amine hydrochloride.

**9.** A pharmaceutical composition comprising a compound of any one of claims 1 to 8 or a pharmaceutically acceptable salt thereof together with a pharmaceutically acceptable carrier.

**10.** A compound as claimed in any one of claims 1 to 8 for use in a method for the therapeutic treatment of the human or animal body.

**11.** A compound as claimed in any one of claims 1 to 8 as a serotonin agonist.

**12.** A process for the preparation of a compound according to claim 1, which comprises

a) condensing a compound of formula

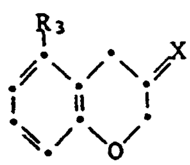

(II),

wherein $R_3$ is as defined above and X is oxygen or reactive esterified hydroxy together with hydrogen, with di-n-propylamine, or
b) alkylating a compound of formula

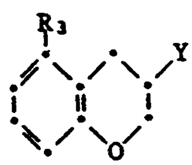

(IV),

wherein $R_3$ is as defined above and Y is $-NH_2$ or $-NH(CH_2CH_2CH_3)$, with a reactive ester of n-propanol or with n-propionaldehyde under reducing conditions, or
c) cleaving a compound of formula

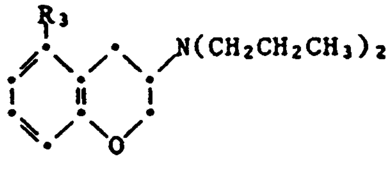

(VI),

wherein $R_3$ is etherified hydroxy, to produce a compound wherein $R_3$ is hydroxy, and/or converting a compound of formula I wherein $R_3$ is hydroxy by etherification into a compound of formula I wherein

$R_3$ is methoxy or ethoxy, or
d) reducing a compound of formula

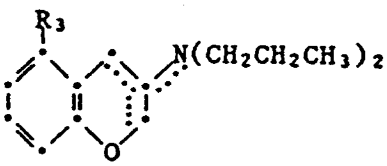

(VII),

wherein $R_3$ is as defined above and the dotted bond lines are a double bond in one of the positions indicated, or
e) reducing a compound of formula

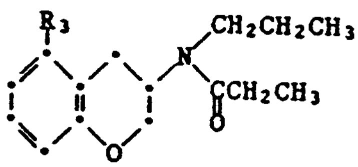

(VIII)

wherein $R_3$ is as defined above,

and, if desired, protecting reactive functional groups intermediately while carrying out one of the said processes and then isolating the free compound of formula I and/or converting an obtainable compound of formula I into a different compound of formula I and/or converting an obtainable free compound into a salt or converting a salt into the free compound or into a different salt and/or resolving a racemate into the optical antipodes.

**Claims for the following Contracting States : ES, GR**

**1.** A process for the preparation of a (+)- or (-)-enantiomer of a compound of formula

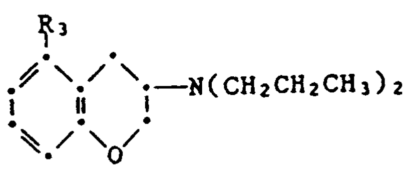

(I)

wherein $R_3$ is methoxy or ethoxy, or a pharmaceutically acceptable salt of that compound, which comprises

a) condensing a compound of formula

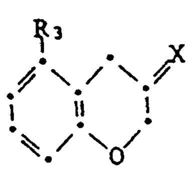

(II),

wherein $R_3$ is as defined above and X is oxygen or reactive esterified hydroxy together with hydrogen, with di-n-propylamine, or
b) alkylating a compound of formula

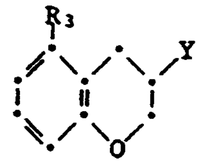

(IV),

wherein $R_3$ is as defined above and Y is $-NH_2$ or $-NH(CH_2CH_2CH_3)$, with a reactive ester of n-propanol or with n-propionaldehyde under reducing conditions, or
c) etherifying a compound of formula

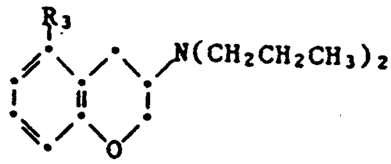

(VI),

wherein $R_3$ is hydroxy, or
d) reducing a compound of formula

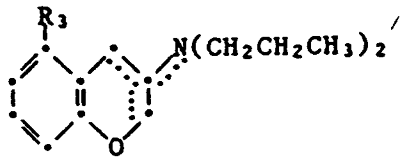

(VII),

wherein $R_3$ is as defined above and the dotted bond lines are a double bond in one of the positions indicated, or
e) reducing a compound of formula

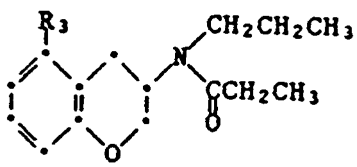

(VIII)

wherein $R_3$ is as defined above,

and, if desired, protecting reactive functional groups intermediately while carrying out one of the said processes and then isolating the free compound of formula I and/or converting an obtainable compound of formula I into a different compound of formula I and/or converting an obtainable free compound into a salt or converting a salt into the free compound or into a different salt and/or resolving a racemate into the optical antipodes.

**2.** A process according to claim 1 for the preparation of the (+)-enantiomer of the compound of formula I wherein $R_3$ is methoxy, or a pharmaceutically acceptable salt of that compound.

**3.** A process according to claim 1 for the preparation of the (-)-enantiomer of the compound of formula I wherein $R_3$ is methoxy, or a pharmaceutically acceptable salt of that compound.

**4.** A process according to claim 1 for the preparation of the (+)-enantiomer of the compound of formula I wherein $R_3$ is ethoxy, or a pharmaceutically acceptable salt of that compound.

**5.** A process according to claim 1 for the preparation of the (-)-enantiomer of the compound of formula I wherein $R_3$ is ethoxy, or a pharmaceutically acceptable salt of that compound.

6. A process according to claim 1 for the preparation of (+)-5-ethoxy-N,N-di-n-propyl-3,4-dihydro-2H-[1]-benzopyran-3-amine hydrochloride.

7. A process for the preparation of a pharmaceutical composition, which comprises mixing a compound obtainable in accordance with any one of claims 1 to 6 with a carrier.

**Revendications**
**Revendications pour les Etats contractants suivants : DE, GB, FR, IT, NL, SE, LI, CH, BE, AT, LU**

1. Enantiomères (+) et (-) des composés de formule

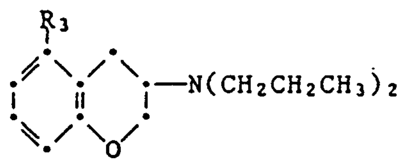

(I)

où $R_3$ représente l'hydroxy, le méthoxy ou l'éthoxy, et les sels pharmaceutiquement acceptables de ces composés.

2. L'énantiomère (+) du composé de formule I selon la revendication 1, où $R_3$ représente l'hydroxy et les sels pharmaceutiquement acceptables de ce composé.

3. L'énantiomère (-) du composé de formule I selon la revendication 1, où $R_3$ représente l'hydroxy et les sels pharmaceutiquement acceptables de ce composé.

4. L'énantiomère (+) du composé de formule I selon la revendication 1, où $R_3$ représente le méthoxy et les sels pharmaceutiquement acceptables de ce composé.

5. L'énantiomère (-) du composé de formule I selon la revendication 1, où $R_3$ représente le méthoxy et les sels pharmaceutiquement acceptables de ce composé.

6. L'énantiomère (+) du composé de formule I selon la revendication 1, où $R_3$ représente l'éthoxy et les sels pharmaceutiquement acceptables de ce composé.

7. L'énantiomère (-) du composé de formule I selon la revendication 1, où $R_3$ représente l'éthoxy et les sels pharmaceutiquement acceptables de ce composé.

8. Le chlorhydrate de la (+)-5-éthoxy-N,N-di-n-propyl-3,4-dihydro-2H-[1]-benzopyranne-3-amine.

9. Préparations pharmaceutiques contenant les composés des revendications 1 à 8 ou l'un de leurs sels pharmaceutiquement acceptables en association avec un matériau de support pharmaceutiquement approprié.

10. Les composés cités dans les revendications 1 à 8 destinés à être utilisés dans un procédé pour le traitement thérapeutique du corps humain ou animal.

11. Les composés cités dans les revendications 1 à 8 comme agonistes de la sérotonine.

12. Procédé pour la préparation des composés selon la revendication 1, caractérisé
a) en ce que l'on condense un composé de formule

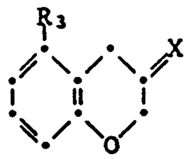

(II),

où $R_3$ a les significations précitées et X représente l'oxygène ou un hydroxy estérifié, réactif, ensemble avec l'hydrogène, avec la di-n-propylamine ou

b) en ce que l'on alcoyle un composé de formule

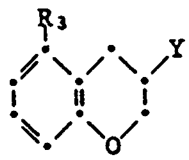

(IV),

où $R_3$ a les significations précitées et Y représente $-NH_2$ ou $-NH(CH_2CH_2CH_2)$, avec un ester réactif du n-propanol ou avec l'aldéhyde n-propionique en respectant des conditions réductrices ou

c) en ce que l'on clive un composé de formule

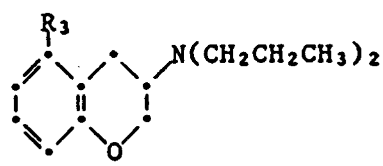

(VI),

où $R_3$ représente un hydroxy éthérifié, pour préparer un composé où $R_3$ représente l'hydroxy et/ou en ce que l'on transforme un composé de formule I où $R_3$ représente l'hydroxy, par éthérification en un composé de formule I où $R_3$ représente le méthoxy ou l'éthoxy ou

d) en ce que l'on réduit un composé de formule

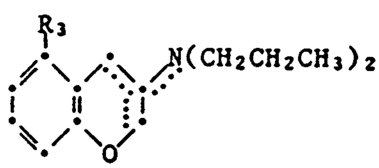

(VII),

où $R_3$ a les significations précitées et où les liaisons en pointillés représentent une liaison double dans l'une des positions représentées ou

e) en ce que l'on réduit un composé de formule

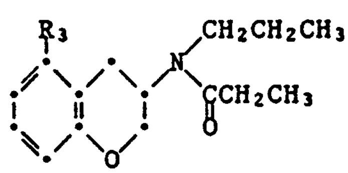

(VIII),

où $R_3$ a les significations précitées,

et, si désiré, en ce que l'on protège temporairement les groupes fonctionnels, réactifs, lors de l'exécution de l'un des procédés précités et en ce que l'on isole ensuite le composé libre de formule I et/ou en ce que l'on transforme le composé libre de formule I obtenu en un autre composé de formule I et/ou en ce que l'on transforme le composé libre obtenu en un sel ou le sel en un composé libre ou en un autre sel et/ou en ce que l'on sépare un racémate en antipodes optiques.

**Revendications pour les Etats contractants suivants : ES, GR**

**1.** Procédé pour la préparation des énantiomères (+) et (-) des composés de formule

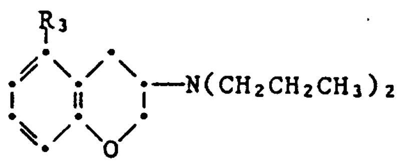

(I)

où $R_3$ représente le méthoxy ou l'éthoxy et des sels pharmaceutiquement acceptables de ces composés, caractérisé

a) en ce que l'on condense un composé de formule

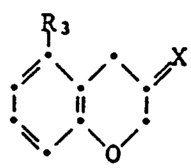

(II),

où $R_3$ a les significations précitées et X représente l'oxygène ou un hydroxy estérifié, réactif, ensemble avec l'hydrogène, avec la di-n-propylamine ou

b) en ce que l'on alcoyle un composé de formule

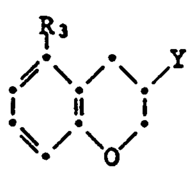

(IV),

où $R_3$ a les significations précitées et Y représente $-NH_2$ ou $-NH(CH_2CH_2CH_3)$, avec un ester réactif du n-propanol ou avec l'aldéhyde n-propionique en respectant des conditions réductrices ou

c) en ce que l'on éthérifie un composé de formule

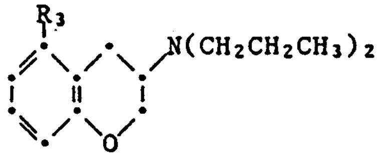

(VI),

où $R_3$ représente l'hydroxy ou

d) en ce que l'on réduit un composé de formule

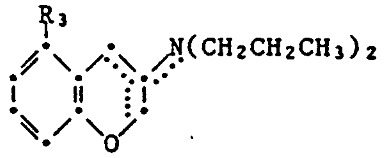

(VII),

où $R_3$ a les significations précitées et où les liaisons en pointillés représentent une liaison double dans l'une des positions représentées ou

e) en ce que l'on réduit un composé de formule

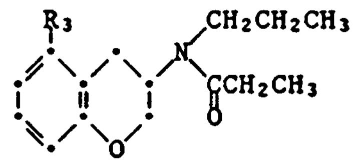

(VIII),

où $R_3$ a les significations précitées,

et, si désiré, en ce que l'on protège temporairement les groupes fonctionnels, réactifs, lors de l'exécution de l'un des procédés précités et en ce que l'on isole ensuite le composé libre de formule I et/ou en ce que l'on transforme le composé libre de formule I obtenu en un autre composé de formule I et/ou en ce que l'on transforme le composé libre obtenu en un sel ou le sel en un composé libre ou en un autre sel et/ou en ce que l'on sépare un racémate en antipodes optiques.

**2.** Procédé selon la revendication 1 pour la préparation de l'énantiomère (+) du composé de formule I où $R_3$ représente le méthoxy et des sels pharmaceutiquement acceptables de ce composé.

**3.** Procédé selon la revendication 1 pour la préparation de l'énantiomère (-) du composé de formule I où $R_3$ représente le méthoxy et des sels pharmaceutiquement acceptables de ce composé.

**4.** Procédé selon la revendication 1 pour la préparation de l'énantiomère (+) du composé de formule I où $R_3$ représente l'éthoxy et des sels pharmaceutiquement acceptables de ce composé.

**5.** Procédé selon la revendication 1 pour la préparation de l'énantiomère (-) du composé de formule I où $R_3$ représente l'éthoxy et des sels pharmaceutiquement acceptables de ce composé.

**6.** Procédé selon la revendication 1 pour la préparation du chlorhydrate de la (+)-5-éthoxy-N,N-di-n-propyl-3,4-dihydro-2H-[1]-benzopyranne-3-amine.

**7.** Procédé pour l'obtention des préparations pharmaceutiques, caractérisé en ce que l'on mélange un composé pouvant être obtenu selon les revendications 1 à 6 avec un matériau de support.